# EUROPÄISCHE PATENTANMELDUNG

(11) **EP 3 556 460 A1**
(43) Veröffentlichungstag der Anmeldung: **23.10.2019**
(21) Anmeldenummer: 18168501.7
(22) Anmeldetag: 20.04.2018
(51) Int. Cl.: B01J 8/00, B01J 19/24, B01J 8/02, C07C 31/04

(54) **REAKTOR ZUR UMSETZUNG GLEICHGEWICHTSREDUZIERTER REAKTIONEN**

(71) Anmelder: Siemens Aktiengesellschaft, 80333 München (DE)
(72) Erfinder: Baldauf, Manfred, 91056 Erlangen (DE); Glöser, Lukas, 91052 Erlangen (DE); Stark, Katharina, 91052 Erlangen (DE); Tremel, Alexander, 91096 Möhrendorf (DE)

(57) **Zusammenfassung**

Die Erfindung betrifft einen Reaktor zur Umsetzung gleichgewichtsreduzierter Reaktionen umfassend ein rohrförmig ausgestaltetes Reaktorgehäuse (4)
- in dem eine, zum Durchfluss eines flüssigen Absorptionsmittels (6) dienende, erste Zone (8) angeordnet ist, die sich in Rohrlängsrichtung (10) erstreckt und
- in dem eine sich ebenfalls in Rohrlängsrichtung (10) erstreckende zweiten Zone (12) zur Aufnahme eines Katalysatormaterials (14) angeordnet ist. Die Erfindung zeichnet sich dadurch aus, dass
- die erste Zone (8) und die zweite Zone (12) durch eine gasdurchlässigen Trennzone (16) getrennt sind,
- die Trennzone (16) eine mechanisch selbstragende Struktur (26) aufweist und
- das Aspektverhältnis des rohrförmigen Reaktorgehäuses (4) entlang einer Reaktionszone (28) größer als 6 ist.

## Beschreibung

Die Erfindung betrifft einen Reaktor zur Umsetzung gleichgewichtsreduzierter Reaktionen nach dem Oberbegriff des Patentanspruchs 1 sowie einen Reaktorbündel nach Anspruch 13 und ein Verfahren zum Betreiben eines Reaktors nach Anspruch 14 und ein Kraftwerkssystem umfassend einen Stromgenerator und einen Reaktor gemäß Anspruch 15.

Fossile Energieträger verursachen Kohlendioxidemissionen, die die Einhaltung globaler Klimaschutzziele erschweren. Aus diesem Grund wird der Ausbau erneuerbarer Energien vorangetrieben. Allerdings ist die Erzeugung regenerativen Stroms deutlich regionalen sowie zeitlichen Schwankungen unterworfen.

Beispielsweise wird bei sonnigen oder windigen Tagen durch Photovoltaik bzw. Windkraftanlagen Strom kostengünstig produziert. Derzeit wird nach wirtschaftlichen Ansätzen gesucht, diesen Strom auch über den Elektrizitätssektor hinaus sinnvoll zu nutzen und beispielsweise hieraus chemische Wertprodukte zu erzeugen. Eine Möglichkeit ist dabei die elektrochemische Umwandlung von Wasser in Wasserstoff und Sauerstoff. Der erzeugte Wasserstoff kann dann mit dem klimaschädlichen Kohlendioxid als Startmolekül bzw. Edukt reagieren, wodurch gleichzeitig Kohlendioxidemission verringert werden würde. Das relativ einfach verfügbare Kohlendioxid kann damit als kostengünstige Kohlenstoffquelle beispielsweise bei der Herstellung von Methanol als ein mögliches Produkt einer einstufigen Synthese von Kohlenstoffdioxid und Wasserstoff nach der Reaktionsgleichung

C0₂ + 3H₂ -> CH₃0H + H₂0

reagieren. Nachteilig an der Synthese von Methanol aus Kohlendioxid und Wasserstoff sind die niedrigen Gleichgewichtsumsätze, die in etwa nur 20 % bei 50 bar und 250°C betragen.

Daher wird in herkömmlichen Syntheseanlagen ein großer Teil der gasförmigen Edukte im Kreis geführt, was zu erheblichen Druckverlusten im Reaktor und in Rohrleitungen führt und wodurch ein erheblicher Energieeintrag in Form von Kompressionsleistung und Wärmeleistung erfolgen muss. Desweiteren ist ein Gasrecycling wenig geeignet für einen dynamischen Betrieb einer Reaktionsanlage und kann deshalb auch schlecht an unregelmäßig auftretenden elektrischen Energiemengen angepasst werden, wie sie durch den fluktuierende Erzeugung vorherrschen.

Ein Ansatz zur Umsetzung von gleichgewichtslimitierten Reaktionen wird bereits in der WO 2017212016 A1 beschrieben. Hierbei kommt ein Rührkesselreaktor zum Einsetz, in dem ein Absorptionsmittel in einem unteren Bereich des Reaktors vorliegt und Eduktgase durch eine Katalysatoranordnung geleitet werden, wobei Produkte von dem Katalysator weit getrennt vorliegenden Absorptionsmittel aufgenommen werden. Eine derartige Anlage ist durchaus zweckdienlich, sie ist aber durch die Rühranordnung technisch aufwendig und weist Einschränkungen in der dynamischen Abfuhr des Produktes und der dynamischen Zufuhr von elektrischer Energie auf.

Die Aufgabe der Erfindung besteht darin, einen Reaktor zur Umsetzung gleichgewichtslimitierter Reaktionen bereitzustellen, die gegenüber dem Stand der Technik mit einem geringeren technischen Aufwand realisierbar sind und dabei eine kontinuierlichen Prozess erlauben, der gut dynamisch regelbar ist.

Die Lösung der Aufgabe besteht in einem Reaktor zur Umsetzung gleichgewichtslimitierter Reaktionen mit den Merkmalen des Anspruchs 1, in einem Reaktorbündel nach Anspruch 13, einem Verfahren zum Betreiben eines Reaktors nach Anspruch 14 sowie in einem Kraftwerkssystem mit einem Stromgenerator mit den Merkmalen des Anspruchs 15. Der erfindungsgemäße Reaktor zur Umsetzung gleichgewichtsreduzierter Reaktionen gemäß Anspruch 1 umfasst ein rohrförmig ausgestaltetes Reaktorgehäuse 4. In diesem ist zum Durchfluss eines flüssigen Absorptionsmittels eine erste Zone angeordnet, die sich in Rohrlängsrichtung erstreckt. Ferner umfasst das rohrförmige Reaktorgehäuse eine zweite Zone zur Aufnahme eines Katalysatormaterials, wobei die zweite Zone sich ebenfalls entlang der Rohrlängsrichtung erstreckt. Die Erfindung zeichnet sich dadurch aus, dass die erste Zone und die zweite Zone durch eine gasdurchlässige Trennzone getrennt sind, wobei die Trennzone eine mechanisch selbsttragende Struktur aufweist. Ferner ist die Erfindung dadurch gekennzeichnet, dass das rohrförmige Reaktorgehäuse ein Aspektverhältnis aufweist, das größer als sechs ist, was bedeutet, dass die Länge des Rohres mindestens sechs Mal größer ist als der Innendurchmesser des Rohres.

Der erfindungsgemäß Reaktor ermöglicht einen kontinuierlichen Durchfluss von Absorptionsflüssigkeit in direkter Umgebung und entlang einer sich längs des rohrförmigen Reaktors erstreckenden Katalysatormaterials, sodass kontinuierlich ein Produkt, das an der Katalysatoroberfläche entsteht, allein getrennt durch die mechanisch tragende Trennzone aufgenommen werden kann. Das durch das Absorptionsmittel aufgenommene Produkt wird kontinuierlich aus der Reaktionszone im Bereich des Katalysatormaterials abgeführt, weshalb wieder ein neues Produkt aufgrund des sich neu einstellenden Gleichgewichts entstehen kann. Der Reaktor gemäß Anspruch 1 kommt nahezu ohne bewegliche Teile bei der Reaktionsführung aus, weshalb er einem geringeren Verschleiß unterzogen ist und dabei einen kostengünstigen technischen Aufbau erlaubt. Je nach Durchflussrate des Absorptionsmittels entlang der ersten Zone und je nach Einfüllen von Eduktgasen in die zweite Zone, kann der Reaktionsablauf und der Umsetzungsgrad reguliert werden. Bei geringen zur Verfügung stehenden Energiemengen kann die Reaktion auf ein Minimum heruntergefahren werden, bei einem Überschuss an elektrischer Energie kann die Reaktion im Reaktor entsprechend und kontinuierlich verstärkt werden. Es handelt sich um einen kontinuierlichen Prozess, der insbesondere durch den kontinuierlichen Durchlauf des Absorptionsmittels durch die erste Zone geregelt ist.

Bei dem Begriff rohrförmig wird dabei ein langgestrecktes innen hohles Gebilde verstanden, das ein Aspektverhältnis aufweist, dass größer als sechs ist, bevorzugt größer als acht, besonders bevorzugt größer als zwölf ist. Bevorzugt ist der Querschnitt des rohrförmigen Reaktorgehäuses rund oder oval, wobei aber auch andere, wie beispielsweise rechteckige oder quadratische Querschnitte unter rohrförmig verstanden werden. Unter dem Begriff Trennzone wird ein Bereich verstanden, der zumindest eine mechanisch selbsttragende Struktur aufweist, die insbesondere dazu dient, das Katalysatormaterial von der ersten Zone nämlich der vom Absorptionsmittel durchflossenen Zone zu trennen, da die Wirkungsweise des Katalysatormaterials bei Kontakt mit dem Absorptionsmittel negativ beeinflusst werden würde. Die Trennzone an sich kann dabei mehrere Maßnahmen mechanischer und/oder chemischer Art umfassen, beispielsweise kann sie hydrophobe Schichten umfassen oder Abstandshalter, die ein mechanisch selbsttragendes Netzgebilde oder eine Folienstruktur bzw. eine Membran mit dem Katalysatormaterial von der ersten Zone beabstandet halten.

In einer bevorzugten Ausgestaltungsform des Reaktors ist die erste Zone an einer Innenwand des rohrförmigen Reaktorgehäuses angeordnet. An der Innenwand entlang kann die Absorptionsflüssigkeit an dem Reaktorgehäuse, bevorzugt angetrieben durch die Schwerkraft entlang fließen und dabei Reaktionsprodukte, die bei den Umgebungsbedingungen gasförmig sind, absorbieren.

Bevorzugt umgibt die erste Zone ausgehend von der Innenwand des rohrförmigen Reaktors die zweite Zone konzentrisch. Das bedeutet, dass die zweite Zone mit dem Katalysatormaterial im Zentrum des rohrförmigen Reaktors angeordnet ist und diese zweite Zone von der ersten Zone konzentrisch umgeben wird. Der Begriff konzentrisch schließt in analoger Weise auch nicht kreisrunde, also auch rechteckige Querschnitte mit ein. Dies ermöglicht einen Reaktoraufbau, der technisch mit geringem Aufwand realisierbar ist.

In einer Ausgestaltungsform der Erfindung erstreckt sich dabei die erste Zone und die zweite Zone entlang einer gesamten Reaktionszone des rohrförmigen Reaktorgehäuses in Rohrlängsrichtung und sie sind dabei entlang der Reaktionszone getrennt durch die Trennzone. Dieser langgezogene Aufbau mit einem kontinuierlichen Kontakt zwischen erster und zweiter Zone, lediglich getrennt durch die Trennzone, erlaubt einen besonders kontinuierlichen Verlauf der Reaktion und ein kontinuierlichen Durchlauf des Absorptionsmittels durch den Reaktor. Dabei ist es zweckmäßig, dass der Reaktor nicht vertikal angeordnet ist, dass er mindestens einen Winkel von 10 Grad, bevorzugt mehr, im Idealfall 90 Grad zur Vertikalen aufweist. Durch die Wahl des Neigungswinkels des Reaktors kann die Strömungsgeschwindigkeit des Absorptionsmittels reguliert werden, und somit die Reaktion an sich beeinflusst werden.

Die erste Zone des Reaktors umfasst bevorzugt eine poröse Struktur, die wiederum bevorzugt eine hydrophile Überfläche, d. h. insbesondere eine hydrophile Oberfläche gegenüber der Absorptionsflüssigkeit aufweist. Unter hydrophil wird dabei verstanden, dass der Benetzungswinkel zwischen der Oberfläche und der entsprechenden Flüssigkeit kleiner als 90 Grad ist. Im Gegensatz dazu ist ein Benetzungswinkel über 90 Grad hydrophob.

Eine poröse Struktur mit einer bevorzugt hydrophilen Oberfläche erlaubt das langsame gleichmäßige und gezielte Durchfließen der Absorptionsflüssigkeit durch die erste Zone, wobei durch die poröse Struktur eine möglichst große Oberfläche geschaffen wird, an der das Absorptionsmedium entlang fließen kann.

Ferner ist in einer bevorzugten Ausgestaltungsform die mechanisch selbsttragende Struktur der Trennzone mit einer hydrophoben Schicht versehen, das bewirkt wiederum, dass flüssiges Absorptionsmittel daran gehindert wird, in die zweite Zone einzudringen und somit daran gehindert wird, mit den Katalysatormaterial in Kontakt zu kommen.

Die mechanisch selbsttragende Struktur, sei es in Form eines keramischen Mediums oder sei es in Form eines Netzes oder eines Gewebes, das in der Form ausgestaltet ist, dass es den Prozesstemperaturen standhält, hat dabei eine Porosität, die größer ist als die Porosität der porösen Struktur der ersten Zone. Dies erlaubt den Produkten ein Durchdringen der Trennzone und eine anschließende Aufnahme der Produkte durch das Absorptionsmittel.

In einer weiteren Ausgestaltungsform der Erfindung umfasst der Reaktor ein Absorptionsmitteleinlass und ein Absorptionsmittelauslass zur kontinuierlichen Durchführung des Absorptionsmittels durch die erste Zone und umfasst ein Eduktgaseinlass zum Einlass des Eduktgases in die zweite Zone. Insbesondere ist dabei bevorzugt kein Eduktgasauslass aus der zweiten Zone vorgesehen, das Eduktgas bleibt bevorzugt stationär in der zweiten Zone bis zu seiner vollständigen Umwandlung bestehen. Ein derartiger Aufbau der zweiten Zone wird auch als Dead-End-Bauweise bezeichnet.

Ein weiterer Bestandteil der Erfindung ist ein Reaktorbündel umfassend mindestens zwei Reaktoren nach einen der vorhergehenden Ansprüche. Der Reaktorbündel zeichnet sich dadurch aus, dass die mindestens zwei Reaktoren gemeinsam in einem Kühlflüssigkeitsbehälter angeordnet sind.

Ein weiterer Bestandteil der Erfindung ist ein Verfahren zum Betreiben eines Reaktors oder Reaktorbündels aus den vorhergehenden Ansprüchen, wobei sich das Verfahren dadurch zeichnet, dass ein Absorptionsmittel kontinuierlich in die erste Zone eingeleitet wird und an einem Ende des rohrförmigen Reaktorgehäuses wieder ausgeleitet wird. Ferner wird das gasförmige Edukt in die zweite Zone eingeleitet, wobei es an einer Oberfläche des dort, also in der zweiten Zone befindlichen Katalysatormaterials zumindest teilweise in mindestens ein Produkt reagiert. Das so reagierte Produkt wird anschließend durch die Trennzone in die erste Zone geführt und von dem Absorptionsmittel absorbiert und das Produkt wird mit dem Absorptionsmittel aus dem Reaktor ausgeleitet.

Sowohl der Reaktorbündel als auch das Verfahren zum Betreiben eines Reaktors weisen dieselben Vorteile auf, die bereits bezüglich des Reaktors gemäß Anspruch 1 dargelegt sind. Insbesondere werden durch das Verfahren ein kontinuierlicher Verlauf und eine gute Regelbarkeit der Reaktion bei geringem technischen Aufwand bewirkt. Dies ist auch auf einen Verzicht bewegte mechanischer Teile zurückzuführen was sowohl das Verfahren als auch den Reaktorbündel auszeichnet.

Ferner ist es in einer Ausgestaltungsform zweckmäßig, eine Kombination zwischen dem Reaktor und einer Stromerzeugungseinheit herzustellen und somit ein Kraftwerksystem zu generieren, das insgesamt den beschriebenen Vorteil des Reaktors nutz, um überschüssige Energie direkt in chemische Wertstoffe umzuwandeln. Darunter wird auch verstanden, die überschüssige Energie zur Erzeugung der Edukte zu verwenden, beispielsweise für den Betrieb eines Elektrolyseurs zur Herstellung von Wasserstoff. Dabei kann die bereits beschriebene Methanol-Reaktion bevorzugt zum Einsatz kommen. Die Stromerzeugungseinheit ist dabei in der Regel ein Stromgenerator, es können jedoch auch Photovoltaikanlagen als Stromerzeugungseinheit dienen. Eine Kombination der Stromerzeugungseinheit und des Reaktors in dem genannten Kraftwerkssystem ist insbesondere dann zweckmäßig, wenn durch das Kraftwerk, dass als zentrale Einheit die Stromerzeugungseinheit umfasst, Energie erzeugt wird, die auf dem freien Strommarkt einen zu geringen Preis erzielen würde. Ab einer bestimmten Preisgrenze ist dann die technische Umwandlung der elektrischen Energie in einen chemischen Wertstoff zweckmäßig. Dabei kann das Kraftwerk unterschiedlicher Gestalt sein, es kann sich dabei um eine Anlage zur Erzeugung regenerativer Energie, wie z. B. um eine Windkraftanlage handel, ebenso kann es sich um ein klassische, insbesondere fossiles Großkraftwerk, wie z. B. ein Gaskraftwerk handeln.

Weitere Ausgestaltungsformen der Erfindung und weitere Merkmale werden anhand der folgenden Figuren näher erläutert. Dabei zeigen
- Figur 1: eine schematische Darstellung und Ausschnitt aus einem Reaktor mit einem rohrförmigen Gehäuse und
- Figur 2: ein Reaktorbündel aus einer Mehrzahl von Reaktoren gemäß Figur 1.

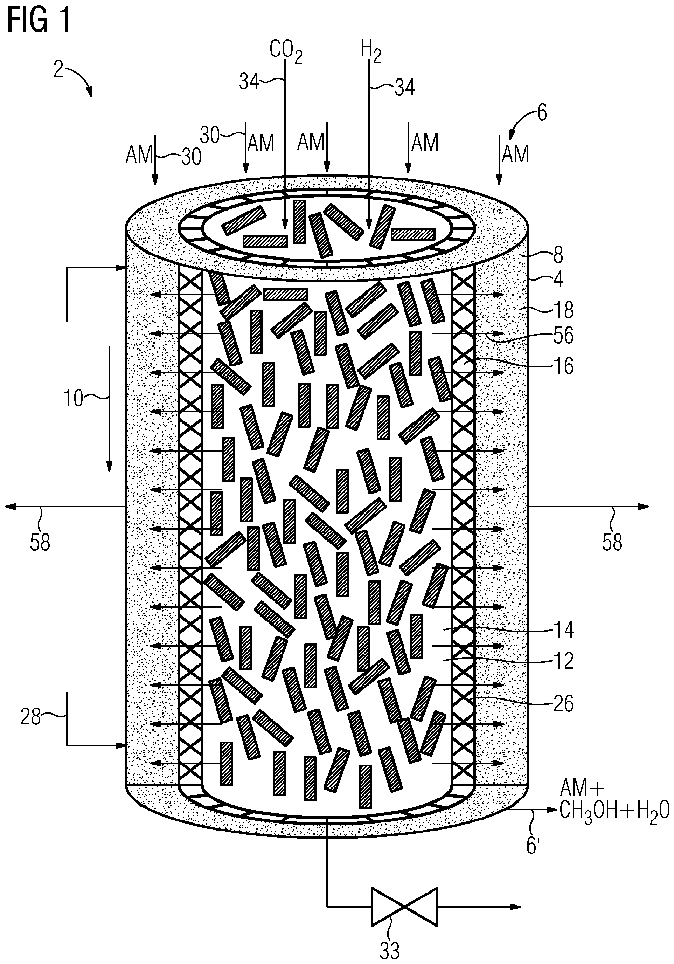

In Figur 1 ist ein Reaktor 2 dargestellt, der ein rohrförmiges Reaktorgehäuse 4 umfasst. In dem rohrförmigen Reaktorgehäuse 4 ist an einer Innenwand 20 des Reaktorgehäuses 4 eine erste Zone 8 angeordnet, die zum Durchlauf eines Absorptionsmittels (AM) 6 dient. Bevorzugt weist diese erste Zone 8 eine poröse Struktur 18 auf, damit das Absorptionsmittel 6 mit einer möglichst großen Oberfläche in Berührung kommt und möglichst viel Absorptionsmittel mit einer großen Oberfläche durch die erste Zone 8 geleitet werden kann. Der Reaktor 2 steht dabei aufrecht, sodass das Absorptionsmittel 6 bevorzugt mithilfe der Schwerkraft durch die erste Zone 8 fließt. Die poröse Struktur 18 erhöht die Oberfläche und somit die Aufnahmefähigkeit des Absorptionsmittels, grundsätzlich wäre es jedoch auch zweckmäßig, das Absorptionsmittel 8 entlang der Innenwand 20 des Reaktorgehäuses 4 langsam fließen zu lassen. Das Absorptionsmittel 6 wird im oberen Bereich des Reaktors in die erste Zone 8 eingespeist, was in Figur 1 nicht näher technisch dargestellt ist.

Konzentrisch umgeben von der ersten Zone 8 befindet sich eine zweite Zone 12 im Zentrum des Reaktorgehäuses 4, wobei in der zweiten Zone 12 im Wesentlichen ein Katalysatormaterial 14 angeordnet ist. Zur Ausgestaltung des Katalysatormaterials gibt es eine Vielzahl zweckmäßigen Möglichkeiten, insbesondere handelt es sich um eine Schüttung, es kann jedoch auch ein poröses Trägermaterial mit einer hohen Oberfläche bereitgestellt werden, an der in dünnen Schichten Katalysatormaterial aufgebracht ist, ähnlich eines Abgaskatalysators aus der Automobiltechnologie. Kostengünstig ist jedoch eine Schüttung von einem Katalysatormaterial, wobei als Katalysator für die Methanolherstellung beispielsweise eine Mischung aus Kupfer, Aluminiumoxid und Zinkoxid zum Einsatz kommt. Korngröße und Konform des geschütteten Materials ist dabei der Prozesstechnik angepasst.

Es hat sich herausgestellt, dass das Absorptionsmittel 6 und das Katalysatormaterial 14 möglichst nicht in Berührung kommen sollten, da sonst die Wirkungsweise des Katalysatormaterials 14 eingeschränkt ist. Aus diesem Grund ist zwischen der zweiten Zone 12 und der ersten Zone 8 eine Trennzone 16 angeordnet, die insbesondere dazu dient, eben diesen Kontakt zwischen Absorptionsmittel 6 und Katalysatormaterial 14 zu verhindern. Damit ist die Trennzone 16 insbesondere durch ihre Funktion bestimmt, sie kann zur Erfüllung dieser Funktion mehrere Bestandteile mit mehreren unterschiedlichen Wirkungsweisen enthalten. Dabei kann die Trennzone 16 beispielsweise ein hier nicht dargestelltes Gewebe, beispielsweise aus Metall, wie Edelstahl oder aus Kohlenstoff oder anderen mineralischen Fasern enthalten, in dem das Katalysatormaterial 14 zusammengehalten ist. Dabei wird eine der wesentlichen Eigenschaften der Trennzone 16 damit beschrieben, dass sie eine mechanisch selbsttragende Struktur 26 umfasst. Die selbsttragende Struktur 26 ist somit beispielsweise in Form eines temperaturbeständigen und chemisch inerten Gewebes erfüllt. Sie kann jedoch auch in Form eines porösen keramischen Topfes ausgestaltet sein. Wichtig ist dabei die physikalische und chemische Trennung zwischen dem Absorptionsmittel 6 und dem Katalysatormaterial 14. Ferner können beispielsweise Abstandshalter, die hier nicht explizit dargestellt sind, an der Innenwand 20 des Reaktorgehäuses 4 angeordnet sein, die nach dieser Definition auch zur Trennzone 16 gehören, und die die selbsttragende Struktur 26, beispielsweise ausgestaltet in Form eines Gewebes von der ersten Zone 8 fernhalten.

In die zweite Zone 12 wird nun ein gasförmiges Edukt 54, das insbesondere bei der Methanolherstellung Kohlendioxid und Wasserstoff umfasst, in die zweite Zone 12 des Reaktors 2 eingeleitet. Bei dem genannten Edukt handelt es sich um ein Ausführungsbeispiel, alternative Eduktgase zur Umsetzung gleichgewichtslimitierter Reaktionen sind hier ebenso zweckmäßig. Das Kohlendioxid und der Wasserstoff reagieren an der Oberfläche des Katalysatormaterials 14 zu Methanol, das bei den vorherrschenden Prozessbedingungen, beispielsweise 50 bar und 250 Grad Celsius gasförmig ist. Das (bei den Prozessbedingungen) gasförmige Produkt Methanol diffundiert dabei durch die selbsttragende Struktur 26 in die erste Zone 8 und wird von dem Absorptionsmittel 6 absorbiert. Das kontinuierlich durch die erste Zone 8 fließende Absorptionsmittel 6 wird aus dem Rohrreaktor wieder ausgeleitet, wobei das beladene Absorptionsmittel nun mit dem Bezugszeichen 6' versehen ist. Das beladene Absorptionsmittel 6' wird in einer nicht dargestellten Vorrichtung beispielsweise durch Absenkung des Drucks entladen und bevorzugt wieder dem Reaktionsprozess zugeführt. Die zweite Zone 12 ist dabei bevorzugt am Ende des Reaktors 2, also am unteren Ende an dem auch das beladene Absorptionsmittel 6' ausgeleitet wird, für das Eduktgas geschlossen. Man nennt dies auch eine Dead-End-Bauweise, was dazu führt, dass das eingebrachte Eduktgas gezwungen ist, vollständig zum Produkt zu reagieren, es wird jedoch an einem Eduktgaseinlass 34 je nach Verbrauch der Edukte 54 kontinuierlich weitere Edukte 54 eingeführt. Ein Durchströmen der zweiten Zone 12 von Eduktgasen wäre zwar technisch möglich, wirtschaftlich jedoch nicht vorteilhaft, weshalb bevorzugt die Dead-End-Bauweise gewählt wird. Es ist lediglich ein Ventil 33 vorgesehen, um ein sogenanntes Purgegas aus der zweiten Zone abzuleiten. Bei dem Purgegas handelt es sich um unerwünschte Gase, insbesondere in Inertgase, wie Stickstoff, die als Abfallprodukte während der Reaktion anfallen. Hierbei wird während der Reaktion das Ventil 33 in regelmäßigen Abständen geöffnet.

Bezüglich des Reaktors 2 beziehungsweise des Reaktorgehäuses 4 soll noch die Rohrlängsrichtung 10 definiert sein, die entlang des Pfeiles 10 erfolgt und durch diesen Charakterisiert ist. Entlang dieser Rohrlängsrichtung 10 befindet sich auch eine Reaktionszone 28, die nahezu über die gesamte Länge des Reaktorgehäuses 22 erfolgt. Bevorzugt ist lediglich im oberen und unteren Bereich zur Versorgung oder Entsorgung von Eduktgas 54 beziehungsweise Zu- und Ableitung des Absorptionsmittels 6 des Reaktorgehäuses gegenüber der Reaktionszone 28 verlängert. Das bedeutet, dass nahezu über die gesamte Länge des Reaktors 2 eine Reaktion stattfindet, was eine besonders gute Raumausnutzung verbunden mit einer kostengünstig technischen Umsetzung bedeutet.

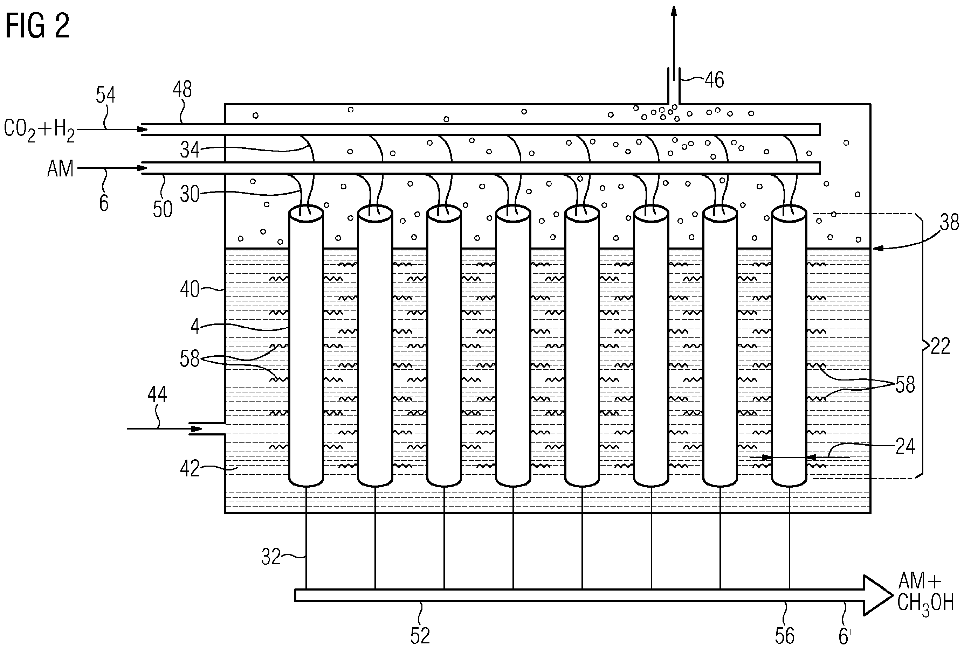

In Figur 2 ist ein Reaktorbündel 38 dargestellt, wobei eine Mehrzahl von Reaktoren 2 in einem Kühlflüssigkeitsbehälter 40 angeordnet sind, in dem sich ebenfalls eine Kühlflüssigkeit 42 befindet. Ferner sind Eduktgaszuleitungen 48 und Absorptionsmittelzuleitung 50 vorgesehen, über die die einzelnen Reaktoren 2 beziehungsweise Reaktorgehäuse 4 mit Edukten 54 beziehungsweise Absorptionsmittel 6 versorgt werden. Dazu weisen die Reaktoren 2 Absorptionsmitteleinlassvorrichtungen 30 auf, über die das Absorptionsmittel 6 in die erste Zone 8 des Reaktors 2 geleitet wird. Ferner weisen die Reaktoren einen Absorptionsmittelauslass 32 auf, in dem das beladene Absorptionsmittel 6' ausgeleitet wird und anschließend, hier nicht näher dargestellt, ein Produkt 56 abgeleitet wird. Ferner ist ein Einlass 44 für das Kühlmittel 42 vorgesehen, wobei Wärme, die sowohl in der Figur 1 als auch in der Figur 2 schematisch mit dem Bezugszeichen 58 dargestellt ist und die bei der Reaktion im Reaktor 2 auftritt, an das Kühlmittel 42 abgegeben wird. Das Kühlmittel 42 verdampft dabei und wird über den Kühlmittelauslass 46 ausgeleitet. Hierbei liegt eine isotherme Prozessführung vor, wobei die Temperatur, die in den Reaktoren vorliegt, durch den Ausgleich von Kühlmittel 42 konstant gehalten wird. Der hierbei entstehende Wasserdampf wird entnommen und es wird neues Kühlmittel 42 zugeführt, was zur konstanten Temperaturführung beiträgt.

Ferner ist es zweckmäßig, wenn ein Reaktor 2 beziehungsweise ein Reaktorbündel 38 mit einer Stromerzeugungseinheit zu einem Kraftwerkssystem kombiniert wird. Aufgrund der Schwankung des Stromangebotes in den Stromnetzen, die insbesondere auf die unterschiedliche Bereitstellung von regenerativen elektrischen Energien zurückzuführen ist, ändert sich der Strompreis in sehr kurzen Zeitintervallen, sodass möglicherweise durch verschiedene Kraftwerke ein kostendeckendes wirtschaftliches Einspeisen von elektrischer Energie nicht möglich ist. In diesem Zusammenhang kann es für alle Kraftwerkstypen, sowohl konventionelle fossile Kraftwerke als auch für regenerative Kraftwerke, wie Solarkraftwerke oder Windkraftwerke zweckmäßig sein, auf eine Einspeisung der erzeugten Energie in das Stromnetz zu verzichten und stattdessen die erzeugt elektrische Energie zur Umwandlung von Produktgasen in einen chemischen Wertstoff, wie das beschriebene Ethanol zu nutzen. Dies kann gegebenenfalls abhängig vom jeweiligen Strompreis und den zu erzielenden Produktpreis einen wirtschaftlichen Vorteil bedeuten. Bei fossilen Kraftwerken kann so gleichzeitig auch der CO2-Ausstoß verringert bzw. die CO2 Bilanz verbessert werden.

Es kann auch für fossile Kraftwerke zweckmäßig sein, diese Kombination zwischen der Stromerzeugungseinheit, insbesondere eines Stromgenerators mit dem beschriebenen Reaktor 2 beziehungsweise dem Reaktorbündel 38 zu nutzen. Das kann dazu führen, dass die Kraftwerkseinheit, beispielsweise ein Gaskraftwerk oder eine Kohlekraftwerk in einem konstanten Leistungsspektrum betrieben werden kann, was die Wirtschaftlichkeit des Kraftwerkes fördert. Die Energieerzeugung des Kraftwerkes muss nicht herabgefahren werden, wenn der Strompreis gering ist, sondern die Energie kann beispielsweise in die beschriebene chemische Reaktion beziehungsweise in den Reaktor 2 beziehungsweise in den Reaktorbündel 38 eingebracht werden. Ferner ist es insbesondere bei der Anwendung der beschriebenen Technologie auf fossile Kraftwerke zweckmäßig, Kohlendioxid, was bei der Verbrennung von fossilen Brennstoffen unweigerlich entsteht, aus den Abgasen des Kraftwerkes abzuzweigen und in die Prozessführung zur Herstellung von Wertgasen, wie beschrieben, in der Methanolsynthese als Eduktgas Kohlendioxid einzuspeisen.

### Bezugszeichenliste

- 2: Reaktor
- 4: Reaktorgehäuse
- 6: Absorptionsmittel (AM)
- 8: erste Zone
- 10: Rohrlängsrichtung
- 12: zweite Zone
- 14: Katalysatormaterial
- 16: Trennzone
- 18: poröse Struktur
- 20: Innenwand Reaktorgehäuse
- 22: Länge Reaktorgehäuse
- 24: Breite Reaktorgehäuse
- 26: mechanisch selbsttragende Struktur
- 28: Reaktionszone
- 30: Absorptionsmitteleinlass
- 32: Absorptionsmittelauslass
- 33: Purgegas Ventil
- 34: Eduktgaseinlass
- 36: Ventil
- 38: Reaktorbündel
- 40: Kühlflüssigkeitsbehälter
- 42: Kühlflüssigkeit
- 44: Einlass Kühlflüssigkeit
- 46: Auslass Kühlflüssigkeit
- 48: Eduktgassammelleitung
- 50: Absorptionsmittelsammelleitung
- 52: Absorptionsmittelsammelleitungauslass
- 54: Edukte
- 56: Produkt
- 58: Wärme

## Patentansprüche

1. Reaktor zur Umsetzung gleichgewichtslimitierter Reaktionen umfassend ein rohrförmig ausgestaltetes Reaktorgehäuse (4)
- in dem eine, zum Durchfluss eines flüssigen Absorptionsmittels (6) dienende, erste Zone (8) angeordnet ist, die sich in Rohrlängsrichtung (10) erstreckt und
- in dem eine sich ebenfalls in Rohrlängsrichtung (10) erstreckende zweite Zone (12) zur Aufnahme eines Katalysatormaterials (14) angeordnet ist,
**dadurch gekennzeichnet, dass**
- die erste Zone (8) und die zweite Zone (12) durch eine gasdurchlässigen Trennzone (16) getrennt sind,
- die Trennzone (16) eine mechanisch selbstragende Struktur (26) aufweist und
- das Aspektverhältnis des rohrförmigen Reaktorgehäuses (4) entlang einer Reaktionszone (28) größer als 6 ist.

2. Reaktor nach Anspruch 1, **dadurch gekennzeichnet, dass** die erste Zone an einer Innenwand (20) des rohrförmigen Reaktorgehäuses (4) angeordnet ist.

3. Reaktor nach Anspruch 1 oder 2, **dadurch gekennzeichnet, dass** die erste Zone (8) konzentrisch um die Innenwand (20) des rohrförmigen Reaktorgehäuses (4) angeordnet ist.

4. Reaktor, nach einem der vorhergehenden Ansprüche, **dadurch gekennzeichnet, dass** die erste Zone (8) die zweite Zone (12), getrennt durch die Trennzone (16) konzentrisch umgibt.

5. Reaktor nach einem der vorhergehenden Ansprüche , **dadurch gekennzeichnet, dass** sich die erste Zone (8) und die zweite Zone (12) entlang der gesamten Reaktionszone (22) des rohrförmigen Reaktorgehäuses (4) in Rohrlängsrichtung (10), getrennt durch die Trennzone (16) erstrecken.

6. Reaktor nach einem der vorhergehenden Ansprüche, **dadurch gekennzeichnet, dass** das rohrförmige Reaktorgehäuse (4) in einer betriebsbereiten Positionierung einen Winkel gegenüber einer Vertikal aufweist, der zwischen 10° und 90° liegt.

7. Reaktor nach einem der vorhergehenden Ansprüche, **dadurch gekennzeichnet, dass** die erste Zone (8) eine poröse Struktur (18) enthält.

8. Reaktor nach Anspruch 7, **dadurch gekennzeichnet, dass** die poröse Struktur (18) in der ersten Zone (8) zumindest teilweise eine hydrophile Oberfläche aufweist.

9. Reaktor nach einem der vorhergehenden Ansprüche, **dadurch gekennzeichnet, dass** die mechanisch selbsttragende Struktur (26) mit einer hydrophoben Schicht versehen ist.

10. Reaktor nach einem der vorhergehenden Ansprüche, **dadurch gekennzeichnet, dass** eine Porosität der selbsttragenden Struktur (26) höher ist als eine Porosität der porösen Struktur (18) der ersten Zone (8).

11. Reaktor nach einem der vorhergehenden Ansprüche, **dadurch gekennzeichnet, dass** die Trennzone (16) mindestens eine hydrophobe Schicht umfasst.

12. Reaktor nach einem der vorhergehenden Ansprüche, **dadurch gekennzeichnet, dass** der Reaktor (2) ein Absorptionsmitteleinlass (30) und einen Absorptionsmittelauslass (32) zur kontinuierlichen Durchführung des Absorptionsmittels (6) durch die erste Zone (8) aufweist und dass die zweite Zone (12) einen Eduktgaseinlass (34) aufweist.

13. Reaktorbündel umfassend mindestens zwei Reaktoren (2) nach einem der Ansprüche 1 bis 11, **dadurch gekennzeichnet, dass** die Reaktoren (2) gemeinsam in einem Kühlflüssigkeitsbehälter (40) angeordnet sind.

14. Verfahren zum Betreiben eines Rohreaktors nach einem der Ansprüche 1 bis 13, oder eines Reaktorbündels nach Anspruch 14, **dadurch gekennzeichnet, dass** ein Absorptionsmittel (6) kontinuierlich in die erste Zone (8) eingeleitet wird und an einem Ende des rohrförmigen Reaktorgehäuses (4) wieder ausgeleitet wird und die gasförmigen Edukte (54) in die zweite Zone (12) eingeleitet werden, an einer Oberfläche des dort befindlichen Katalysatormaterials (14) zumindest teilweise zu mindestens einem Produkt (56) reagiert und das Produkt (56) anschließend durch die Trennzone (16) in die erste Zone (8) gelangt und von dem Absorptionsmittel (6) absorbiert wird und mit diesem aus dem Reaktor (2) ausgeleitet wird.

15. Kraftwerksystem umfassend eine Stromerzeugungseinheit und einen Reaktor nach einem der Ansprüche 1 bis 13, wobei vom Stromgenerator erzeugte elektrische Energie zum Betrieb des Reaktors genutzt wird.
